(19) Europäisches Patentamt
European Patent Office
Office européen des brevets

(11) Publication number: 0 153 678
A1

(12) # EUROPEAN PATENT APPLICATION

(21) Application number: 85101715.2

(22) Date of filing: 15.02.85

(51) Int. Cl.[4]: C 07 D 213/30
C 07 D 213/53, C 07 D 213/50
C 07 D 213/38, C 07 D 213/26
A 61 K 31/44, C 07 D 213/00

(30) Priority: 15.02.84 JP 27952/84

(43) Date of publication of application:
04.09.85 Bulletin 85/36

(84) Designated Contracting States:
AT BE CH DE FR GB IT LI LU NL SE

(71) Applicant: Tanabe Seiyaku Co., Ltd.
No. 21 Dosho-machi 3-chome Higashi-ku
Osaka-shi Osaka-fu(JP)

(72) Inventor: Iwakuma, Takeo
No. 5-7, Fujimi 1-chome
Ageo-shi Saitama-ken(JP)

(72) Inventor: Yamada, Koichiro
No. 823-10, Oaza-kizoro
Kawaguchi-shi Saitama-ken(JP)

(72) Inventor: Sasaki, Yasuhiko
Urawa New Heights 410 No. 85, Jinde
Urawa-shi Saitama-ken(JP)

(72) Inventor: Morita, Takashi
No. 13-49, Oaza-Namiki
Kawagoe-shi Saitama-ken(JP)

(72) Inventor: Uchida, Tomofumi
No. 2-5, Momoyamadai 2-chome
Habikino-shi Osaka-fu(JP)

(74) Representative: Hansen, Bernd, Dr.rer.nat. et al,
Hoffmann, Eitle & Partner Patentanwälte Arabellastrasse
4
D-8000 München 81(DE)

(54) **Cinnamic acid derivatives, processes for the preparation thereof and pharmaceutical compositions.**

(57) Novel cinnamic acid derivatives of the formula:

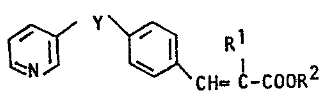

(I)

wherein $R^1$ is a lower alkyl group, $R^2$ is hydrogen atom or a lower alkyl group, and Y is hydroxymethylene, a lower alkoxymethylene, aminomethylene, hydroxyiminomethylene, a halogenomethylene or carbonyl group, and processes for the preparation thereof. Said compounds (I) show in pharmaceutical compositions an inhibitory effect upon thromboxane $A_2$ synthetase and are useful as a platelet aggregation inhibitor and an antithrombotic agent for the prophylaxis and treatment of various thromboses.

EP 0 153 678 A1

41 507 u/wa

0153678

Cinnamic acid derivatives, processes for the preparation thereof and pharmaceutical compositions

This invention relates to novel cinnamic acid derivatives, processes for the preparation thereof and pharmaceutical compositions. More particularly, it relates to cinnamic acid derivatives of the formula:

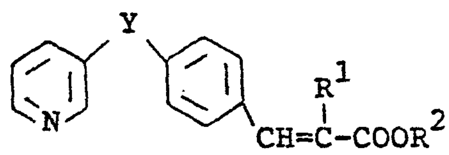

(I)

wherein $R^1$ is a lower alkyl group, $R^2$ is hydrogen atom or a lower alkyl group, and Y is hydroxymethylene, a lower alkoxymethylene, aminomethylene, hydroxyiminomethylene, a halogenomethylene or carbonyl group, and processes for the preparation thereof.

The novel compounds of the formula (I) of the present invention show an inhibitory effect upon thromboxane $A_2$ (abbreviated as $TxA_2$) synthetase and hence are useful as a platelet aggregation inhibitor and an antithrombotic agent. It is known that $TxA_2$ is produced to a large extent by platelets circulating in the blood or when they adhere to the vascular system. $TxA_2$ is also produced by a variety of other cell and tissue types and exerts a variety of biological actions that mediate or exacerbate pathophysiological processes, such as vasospasm, platelet aggregation sometimes leading to thrombosis, etc. Thus, when synthesis of $TxA_2$ in vivo is inhibited, platelet aggregation will be prevented. The compounds of the present invention have

excellent $TxA_2$ synthesis inhibitory activity and hence are useful as a platelet aggregation inhibitor and an antithrombotic agent.

The compounds of the present invention include compounds of the formula (I) wherein $R^1$ is a lower alkyl having 1 to 4 carbon atoms, such as methyl, ethyl, n-propyl, isopropyl, or butyl; $R^2$ is hydrogen atom or a lower alkyl group having 1 to 4 carbon atoms, such as methyl, ethyl, n-propyl, isopropyl, or butyl; and Y is hydroxymethylene, a lower alkoxymethylene having 2 to 5 carbon atoms (e.g. methoxymethylene, ethoxymethylene, n-propoxymethylene, iso-propoxymethylene, n-butoxymethylene or isobutoxymethylene), aminomethylene, hydroxyiminomethylene, a halogenomethylene (e.g. fluoromethylene, chloromethylene, or bromomethylene), or carbonyl group. Preferred compounds are the compounds of the formula (I) wherein $R^1$ is methyl, $R^2$ is hydrogen atom or ethyl, and Y is hydroxymethylene, ethoxymethylene, or chloromethylene. More preferred compounds are the compound of the formula (I) wherein $R^1$ is methyl, $R^2$ is hydrogen atom, and Y is ethoxymethylene.

In the compounds (I) of the present invention, when Y is a substituted methylene group, such as hydroxymethylene, a lower alkoxymethylene, aminomethylene, or a halogenomethylene, there are present two optical isomers wherein the carbon atom in the methylene group is an asymmetric carbon atom. The present invention includes these optical isomers as well as a racemic mixture thereof.

The compounds (I) of the present invention can be

prepared by various processes. For instance, the compounds (I) wherein $R^2$ is hydrogen atom can be prepared by the following Process [A]:

[Process A]

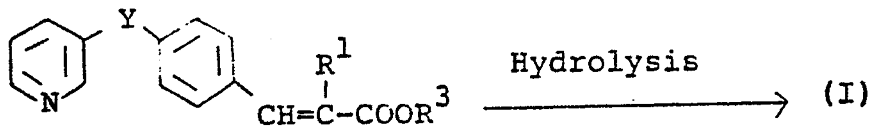


(II)

wherein $R^3$ is a lower alkyl, and $R^1$ and Y are as defined above.

The compounds (I) wherein $R^2$ is a lower alkyl group can be prepared by the following Processes [B] to [G]:

[Process B]

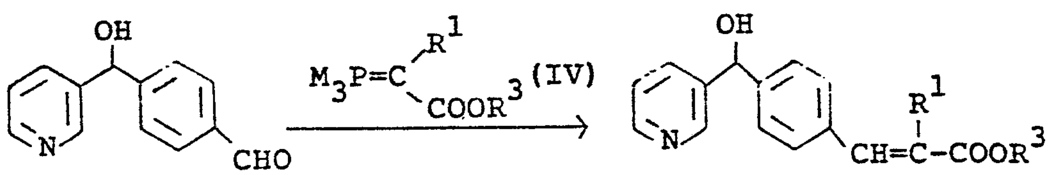


(III) (I-a)

wherein M is phenyl, and $R^1$ and $R^3$ are as defined above.

[Process C]

Compound (I-a)

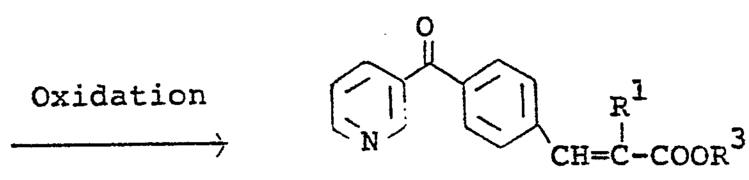


(I-b)

wherein $R^1$ and $R^3$ are as defined above.

[Process D]

Compound (I-b)

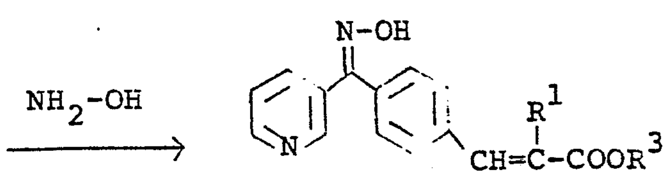


(I-c)

wherein $R^1$ and $R^3$ are as defined above.

[Process E]

Compound (I-c)
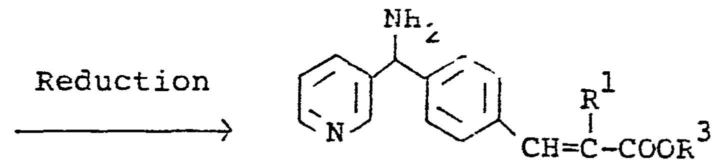


(I-d)

wherein $R^1$ and $R^3$ are as defined above.

[Process F]

Compound (I-a)
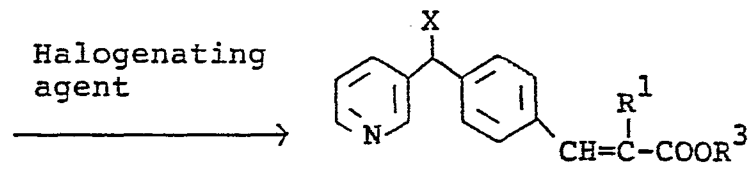


(I-e)

wherein X is a halogen atom, and $R^1$ and $R^3$ are as defined above.

[Process G]

Compound (I-e)
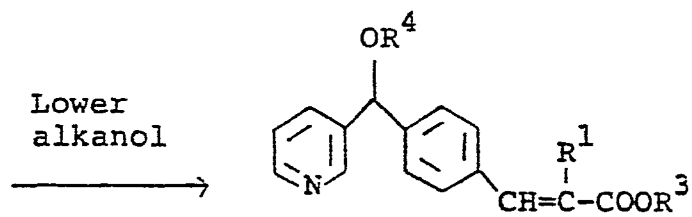


(I-f)

wherein $R^4$ is a lower alkyl group, and $R^1$ and $R^3$ are as defined above.

The above processes are explained in more detail below.

[Process A]:

The hydrolysis of the compounds (II) can be carried out by treating it with an alkali in a suitable solvent. The alkali includes preferably alkali metal hydroxides such as sodium hydroxide, potassium hydroxide, which are usually used in the form of an aqueous solution. The solvent includes, for example, methanol, ethanol, tetra-

hydrofuran, dioxane, isopropanol, monoglyme, or a mixture of one or more of these solvents. The reaction is preferably carried out at a temperature of 20 to 70°C.

[Process B]:

The compounds (I-a) can be prepared by reacting a compound of the formula (III) with a Wittig reagent of the formula (IV). The reaction is readily carried out in a suitable solvent. The solvent includes, for example, chloroform, methylene chloride, tetrahydrofuran, ether, or the like. The reaction is preferably carried out at a temperature of 10 to 20°C.

[Process C]:

The compounds (I-b) can be prepared by oxidizing a compound of the formula (I-a). The oxidation reaction is carried out by oxidizing the compound (I-a) with an oxidizing agent in a suitable solvent. The oxidizing agent includes, for example, barium manganate, active manganese dioxide, potassium permanganate, or the like. The solvent includes chloroform, methylene chloride, tetrahydrofuran, or the like. The reaction is preferably carried out at a temperature of 20 to 60°C.

[Process D]:

The compounds (I-c) can be prepared by reacting a compound of the formula (I-b) with hydroxylamine. The reaction is readily carried out in a suitable solvent. The solvent includes methanol, ethanol, tetrahydrofuran, dioxane, or the like. The reaction is preferably carried out at a temperature of 20 to 70°C.

[Process E]:

The compounds (I-d) can be prepared by reducing a compound of the formula (I-c). The reduction of the compound (I-c) is done with a metal and an acid. The metal includes, for example, zinc, iron, tin, or the like, and the acid includes, for example, acetic acid, propionic acid, hydrochloric acid, diluted sulfuric acid, or the like. In this reaction, the acid can also be used as a solvent, and hence, it is not necessarily required to use any additional solvent. The reaction is preferably carried out at a temperature of 30 to 100°C, particularly 70 to 100°C.

[Process F]:

The compounds (i-e) can be prepared by reacting a compound of the formula (I-a) with a halogenating agent. The halogenating agent includes, for example, thionyl chloride, phosphorus oxychloride, phosphorus pentachloride, or the like, which may also function as a solvent. The reaction is preferably carried out at a temperature of 20 to 50°C.

[Process G]:

The compounds (I-f) can be prepared by reacting a compound of the formula (I-e) with a lower alkanol. The lower alkanol includes, for example, methanol, ethanol, n-propanol, isopropanol, n-butanol, isobutanol, or the like, which may also function as a solvent. The reaction is preferably carried out at a temperature of a boiling point of the solvent.

The compounds (I) of the present invention have

excellent antithrombolic activity as mentioned hereinbefore. For example, when prophylactic effect of a test compound against arachidonic acid-induced pulmonary embolism is estimated by Kohler et al.'s method, said effect of α-methyl-4-(α-hydroxy-3-pyridylmethyl)cinnamic acid and sodium α-methyl-4-(α-ethoxy-3-pyridylmethyl)cinnamate of the present invention is more than 2 times as strong as that of 3-[4-(3-pyridylmethyl)phenyl]-2-methylacrylic acid hydrochloride described in Journal of Medicinal Chemistry vol. 24, No. 10, page 1149 - 1155 (1981).

Moreover, the compounds of the present invention show low toxicity. For example, sodium α-methyl-4-(α-ethoxy-3-pyridylmethyl)cinnamate of the present invention has only about half of the toxicity of sodium salt of the above known compound; i.e. sodium 3-[4-(3-pyridylmethyl)phenyl]-2-methylacrylate.

Hence, the compounds (I) of the invention are useful as an antithrombotic agent for prophylaxis and treatment of various thromboses, such as peripheral artery thrombosis, pulmonary embolism, coronary obstruction, myocardial infarction, cerebral infarction, fugitive cerebral ischemia, etc.

The compounds (I) of the present invention can be used as a medicine in the form of either a free base or a pharmaceutically acceptable salt thereof. The salt includes, for example, inorganic acid addition salts such as hydrochloride, hydrobromide, sulfate, nitrate, etc. and organic acid addition salts such as methanesulfonate,

succinate, maleate, etc., and further alkali metal salts such as sodium salt, potassium salt, lithium salt, etc.

When the compounds (I) or salts thereof are used as a medicine, they can be administered in oral or parenteral route. For oral administration, they are usually used in the form of a solid preparation, such as tablets, pills, powders, capsules, or granules, or a liquid preparation, such as solutions or suspensions. For parenteral administration, they are usually used in the form of an injection. These preparations can be prepared by conventional methods using various conventional pharmaceutically acceptable carriers or diluents.

The daily dose of the compound (I) or a salt thereof may vary depending on the administration route, the age, weight or conditions of patients, and the severity of diseases to be treated. In general, however, a daily dose of said compound (I) or a salt thereof may be about 0.01 to 100 mg/kg/day, and a preferred dose thereof for oral administration may be 0.1 to 100 mg/kg/day, especially 1 to 50 mg/kg/day.

The present invention is illustrated by the following Experiment, Examples and Reference Example, but should not be construed to be limited thereto.

Experiment 1

Prophylactic effect of the compounds against pulmonary embolism induced by arachidonic acid in mice was tested by the method of Kohler et al. (cf. Kohler et al., Wedding & Ellenvogen Thrombosis, 9, pages 67 - 80, 1976).

[Method]:

ddY male mice (weighing about 20 kg, one group: 8 - 10 mice) were fasted one night, and to the animals, test compounds (30 mg/0.25 % CMC 20 ml/kg) were orally administered. After one hour, arachidonic acid (70 mg/10 ml saline solution/kg) was intravenously administered. After the intravenous administration of arachidonic acid, the mice had difficulty in breathing and lay on their stomach without moving and thereafter recovered and started to move. The time from having had difficulty in breathing and laying till starting to move was counted as a recovery time. When the test animal was died, the longest recovery time during the test was counted. The shortening rate of the recovery time was calculated by the following formula for showing the effect of the test compounds. The test compound was evaluated as (-) when the shortening rate was less than 20 %; and evaluated as (±) when it was from 20 % to less than 50 %; and evaluated as (+) when it was more than 50 %.

$$\text{Shortening rate of recovery time (\%)} = \left(1 - \frac{\text{Average recovery time in administration group}}{\text{Average recovery time in non-administration group}}\right) \times 100$$

The test results are shown in Table 1.

Table 1

| Test compounds | Evaluation |
|---|---|
| α-Methyl-4-(α-hydroxy-3-pyridylmethyl)cinnamic acid hydrochlorid | + |
| Sodium α-methyl-4-(α-ethoxy-3-pyridylmethyl)-cinnamate | + |
| Sodium α-methyl-4-(α-methoxy-3-pyridylmethyl)-cinnamate | + |
| Sodium α-methyl-4-(α-n-propoxy-3-pyridylmethyl)-cinnamate | + |
| α-Methyl-4-nicotinoylmethylcinnamic acid hydrochloride | + |
| (known compound) 3-[4-(3-Pyridylmethyl)phenyl]-2-methacrylic acid hydrochloride | ± |

Example 1

To a solution of 4-(α-hydroxy-3-pyridylmethyl)-benzaldehyde (852 mg) in chloroform (25 ml) is added [1-(ethoxycarbonyl)ethylidene]triphenylphosphorane (1.76 g), and the mixture is allowed to stand at room temperature for one day. After the reaction, chloroform is distilled off under reduced pressure, and the residue is purified by silica gel column chromatography (eluent: chloroform) and recrystallized from benzene-n-hexane to give ethyl α-methyl-4-(α-hydroxy-3-pyridylmethyl)cinnamate (1.13 g, yield: 95.3 %) as colorless prisms.

m.p. 148 - 149°C.

Mass (m/e): 297 ($M^+$)

IR (Nujol) $cm^{-1}$: 3125, 1697.

Example 2

Ethyl α-methyl-4-(α-hydroxy-3-pyridylmethyl)cinnamate (1.49 g) is dissolved in chloroform (50 ml), and thereto is added barium manganate (2.9 g). The mixture is refluxed with stirring for one hour. After the reaction, inorganic materials are filtered off, and chloroform solution is concentrated under reduced pressure to give ethyl -methyl-4-nicotinoylcinnamate (1.49 g, yield: quantitative) as pale yellow sticky oily substance.

Mass (m/e): 295 ($M^+$)

IR (Liquid) $cm^{-1}$: 1703, 1658.

Example 3

To a solution of ethyl α-methyl-4-nicotinoylcinnamate (1.48 g) in ethanol (30 ml) is added hydroxylamine hydrochloride (690 mg), and the mixture is refluxed with stirring for one hour. After the reaction, ethanol is distilled off under reduced pressure, and the residue is extracted with chloroform. The extract is washed with saturated aqeuous sodium hydrogen carbonate solution and saturated saline solution successively, and then dried. The chloroform is distilled off under reduced pressure, and the residue is crystallized from isopropanol-n-hexane to give ethyl α-methyl-4-(α-hydroxyimino-3-pyridylmethyl)cinnamate (1.28 g, yield: 82.5 %) as colorless crystals.

Mass (m/e): 310 ($M^+$)

IR (Nujol) $cm^{-1}$: 2400-2800, 1700.

Example 4

To a solution of ethyl α-methyl-4-(α-hydroxyimino-3-pyridylmethyl)cinnamate (1.55 g) in acetic acid (15 ml) is added zinc powder (3 g), and the mixture is stirred at 80°C for 30 minutes. After the reaction, chloroform (50 ml) is added to the reaction mixture, and inorganic materials are filtered off. The chloroform layer is washed with 5 % aqueous ammonia and dried. Chloroform is distilled off under reduced pressure, and the residual crysals are recrystallized from benzene-n-hexane to give ethyl α-methyl-4-(α-amino-3-pyridylmethyl)cinnamate (1.15 g, yield: 77.7 %) as colorless plates.

m.p. 85 - 88°C.

Mass (m/e): 296 ($M^+$)

IR (Nujol) $cm^{-1}$: 3360, 1690.

The ethyl α-methyl-4-(α-amino-3-pyridylmethyl)-cinnamate obtained above is treated with 10 % ethanolic hydrogen chloride solution and then recrystallized from ethanol to give ethyl α-methyl-4-(α-amino-3-pyridylmethyl)-cinnamate dihydrochloride as colorless crystals.

m.p. 229 - 240°C.

IR (Nujol) $cm^{-1}$: 2400-2700, 1700

Elementary analysis for $C_{18}H_{20}N_2O_2.2HCl.\frac{1}{2}H_2O$:

Calcd: C,58.38; H,6.26; N,7.57; Cl,19.15

Found: C,58.23; H,6.01; N,7.55; Cl,18.91

Example 5

Ethyl α-methyl-4-(α-hydroxy-3-pyridylmethyl)-cinnamate (4.43 g) is added to thionyl chloride (50 ml), and the mixture is refluxed with stirring for one hour. After the reaction, excess thionyl chloride is distilled off under reduced pressure, and the residue is crystallized from

ethanol-diethyl ether to give ethyl α-methyl-4-(α-chloro-3-pyridylmethyl)cinnamate hydrochloride (4.88 g) as colorless needles.

m.p. 159 - 162°C.

IR (Nujol) $cm^{-1}$: 2300-2600, 1703.

Example 6

Ethyl α-methyl-4-(α-chloro-3-pyridylmethyl)-cinnamate (3.16 g) is dissolved in ethanol (30 ml), and the mixture is refluxed with stirring for 5 hours. After the reaction, ethanol is distilled off under reduced pressure, and the residue is extracted with chloroform. The extract is washed with saturated aqueous sodium hydrogen carbonate solution and saturated saline solution successively, and dried. The chloroform is distilled off under reduced pressure to give ethyl α-methyl-4-(α-ethoxy-3-pyridylmethyl)-cinnamate (3.24 g) as pale brown sticky oily substance.

Mass (m/e): 325 ($M^+$)

IR ($CHCl_3$) $cm^{-1}$: 1705.

To a solution of the product obtained above in ethanol (30 ml) is added a solution of potassium hydroxide (2.81 g) in water (20 ml), and the mixture is stirred at room temperature overnight. After the reaction, the reaction mixture is adjusted to pH 4 with 10 % hydrochloric acid and then extracted with chloroform. The extract is dried and chloroform is distilled off under reduced pressure to give α-methyl-4-(α-ethoxy-3-pyridylmethyl)cinnamic acid (2.67 g) as brown oily substance.

Mass (m/e): 297 ($M^+$)

IR ($CHCl_3$) $cm^{-1}$: 1682.

Moreover, to a solution of the free carboxylic acid obtained above in ethanol (20 ml) is added 1N sodium hydroxide (9 ml), and the mixture is concentrated under reduced pressure. The residue is crystallized from isopropanol to give sodium α-methyl-4-(α-ethoxy-3-pyridylmethyl)cinnamate (2.39 g) as pale yellow crystals.

m.p. >250°C.

NMR ($D_2O$) δ: 1.15 (t, J=7 Hz, 3H), 1.92 (d, J=2 Hz, 3H), 3.42 (q, J=7 Hz, 3H), 5.38 (s, 1H), 7.0-7.7 (m, 7H), 8.1-8.3 (m, 2H).

Example 7

A solution of ethyl α-methyl-4-(α-chloro-3-pyridylmethyl)cinnamate (632 mg) in methanol (20 ml) is refluxed with stirring for 8 hours. After the reaction, the reaction mixture is concentrated under reduced pressure. The residue is treated with saturated aqueous sodium hydrogen carbonate solution and extracted with chloroform. The chloroform extract is dried and distilled off under reduced pressure. The resulting ethyl α-methyl-4-(α-methoxy-3-pyridylmethyl)cinnamate is dissolved in ethanol (20 ml), and to the mixture is added a solution of potassium hydroxide (850 mg) in water (7 ml), and the mixture is refluxed with stirring for 8 hours. After the reaction, ethanol is distilled off under reduced pressure, and to the residue is added water, and the aqueous solution is adjusted to pH 7 with diluted hydrochloric acid and extracted with chloroform. The aqueous layer is adjusted to pH 4 and extracted with chloroform. The chloroform extracts are

combined and dried. Chloroform is distilled off under reduced pressure, and the residue is solidified from diethyl ether to give α-methyl-4-(α-methoxy-3-pyridylmethyl)cinnamic acid (435 mg) as colorless powder.

Mass (m/e): 283 ($M^+$)

IR (Nujol) $cm^{-1}$: 1680.

To a solution of the free carboxylic acid obtained above in methanol (10 ml) is added 1N aqueous sodium hdyroxide (1.6 ml), and the mixture is concentrated under reduced pressure, and the residue is crystallized from isopropanol-diethyl ether to give sodium α-methyl-4-(α-methoxy-3-pyridylmethyl)cinnamate (332.5 mg) as pale yellow powder.

m.p. > 260°C.

NMR ($D_2O$) δ: 1.94 (d, J=2 Hz, 3H), 3.29 (s, 3H), 5.34 (s, 1H), 7.1-7.9 (m, 7H), 8.3-8.6 (m, 2H).

Example 8

A solution of ethyl α-methyl-4-(α-chloro-3-pyridylmethyl)cinnamate (632 mg) in n-propanol (20 ml) is refluxed with stirring for 5 hours. After the reaction, to the reaction mixture [containing ethyl α-methyl-4-(α-n-propoxy-3-pyridylmethl)cinnamate] is added a solution of potassium hydroxide (850 mg) in a mixture (20 ml) of ethanol-water (1 : 1), and the mixture is refluxed with stirring for 4 hours. After the reaction, the mixture is adjusted to pH 4 with 3N hydrochloric acid, and then the solvent is distilled off under reduced pressure. The residue is extracted with chloroform, and the chloroform layer is dried. Chloroform is distilled off under reduced pressure to give α-methyl-4-

(α-n-propoxy-3-pyridylmethyl)cinnamic acid (610 mg).

Mass (m/e): 311 ($M^+$)

IR ($CHCl_3$) $cm^{-1}$: 1688.

To a solution of the free carboxylic acid obtained above in methanol (10 ml) is added 1N aqueous sodium hdyroxide (2 ml), and the mixture is concentrated under reduced pressure. The residue is crystallized from ethanol-diethyl ether to give sodium α-methyl-4-(α-n-propoxy-3-pyridylmethyl)cinnamate (400 mg) as pale yellow powdery crystals.

m.p. 259 - 263°C. (decomp.)

NMR ($D_2O$) δ: 0.85 (t, J=7 Hz, 3H), 1.1-1.85 (m, 2H), 2.18 (d, J=2 Hz, 3H), 3.36 (t, J=7 Hz, 2H), 5.43 (s, 1H), 7.0-7.9 (m, 7H), 8.3-8.7 (m, 2H).

Elementary analysis for $C_{19}H_{20}O_3NNa.\frac{1}{2}H_2O$:

Calcd: C,66.65; H,6.18; N,4.09

Found: C,67.34; H,6.08; N,3.87

Example 9

In the same manner as described in Example 8, ethyl α-methyl-4-(α-chloro-3-pyridylmethyl)cinnamate (632 mg) and isopropanol (20 ml) are treated to give ethyl α-methyl-4-(α-isopropoxy-3-pyridylmethyl)cinnamate (670 mg). This compound is further treated in the same manner as described in Example 8 to give sodium α-methyl-4-(α-isopropoxy-3-pyridylmethyl)cinnamate (420 mg).

m.p. > 260°C.

NMR ($D_2O$) δ: 1.15 (t, J=6 Hz, 6H), 1.96 (d, J=2 Hz, 3H), 3.3-3.9 (m, 1H), 5.55 (s, 1H), 7.1-7.9 (m, 7H), 8.3-8.6 (m, 2H).

Elementary analysis for $C_{19}H_{20}O_3NNa.\frac{1}{2}H_2O$:

Calcd: C,66.65; H,6.18; N,4.09

Found: C,67.30; H,6.07; N,4.24

Example 10

Ethyl α-methyl-4-(α-hydroxy-3-pyridylmethyl)-cinnamate (700 mg) is dissolved in a mixture of ethanol - tetrahydrofuran (2 : 1) (15 ml) and thereto is added 1N aqueous sodium hydroxide (7.5 ml), and the mixture is stirred at room temperature for one hour and further at 60°C for 2.5 hours. After the reaction, conc. hydrochloric acid (2 ml) is added to the reaction mixture, and the solvent is distilled off under reduced pressure. The residue is added to isopropanol (30 ml), and inorganic materials are filtered off, and thereafter, isopropanol is distilled off under reduced pressure. The crystalline residue is recrystallized from isopropanol-ethanol-diethyl ether to give α-methyl-4-(α-hydroxy-3-pyridylmethyl)cinnamic acid hydrochloride (576 mg, yield: 80 %) as colorless needles.

m.p. 191 - 193°C.

Mass (m/e): 269 ($M^+$)

IR (Nujol) $cm^{-1}$: 1700, 1623.

Example 11

To a solution of ethyl α-methyl-4-nicotinoylmethyl-cinnamate (1.24 g) in ethanol (20 ml) is added 1N aqueous sodium hydroxide (13 ml), and the mixture is stirred at 70°C for 1.5 hour. After the reaction, conc. hydrochloric acid (3.5 ml) is added to the reaction mixture, and the mixture is concentrated under reduced pressure. The residue is

crystallized from water to give α-methyl-4-nicotinoylmethylcinnamic acid hydrochloride (1.03 g, yield: 80.5 %) as pale yellow crystals.

m.p. 233 -236°C.

Mass (m/e): 267 ($M^+$)

IR (Nujol) $cm^{-1}$: 3075, 2700-2300, 1700, 1658.

Example 12

To a solution of ethyl α-methyl-4-(α-amino-3-pyridyl-methyl)cinnamate (296 mg) in ethanol (10 ml) is added a solution of potassium hydroxide (200 mg) in water (2 ml), and the mixture is refluxed with stirring for 1.5 hours. After the reaction, the reaction mixture is acidified with 10 % ethanolic hydrogen chloride solution, and the solvent is distilled off under reduced pressure. The residue is desalted with a non-polar porous resin ("Diaion HP-20", manufactured by Mitsubishi Chemical Ind. Ltd., Japan), and then treated with 10 % ethanolic hydrogen chloride solution. The product is crystallized from isopropanol-diethyl ether to give α-methyl-4-(α-amino-3-pyridylmethyl)cinnamic acid hydrochloride (225 mg, yield: 66 %) as colorless crystals.

m.p. 264 -266°C. (decomp.)

Mass (m/e): 268 ($M^+$)

IR (Nujol) $cm^{-1}$: 2500-2750, 1710.

Example 13

To a solution of ethyl α-methyl-4-(α-hydroxyimino-3-pyridylmethyl)cinnamate (620 mg) in ethanol (10 ml) is added a solution of potassium hydroxide (337 mg) in water (2 ml),

and the mixture is refluxed with stirring for 2 hours. After the reaction, 10 % hydrochloric acid (4 g) is added to the reaction mixture, and the solvent is distilled off under reduced pressure. The residue is desalted with a non-polar porous resin ("Diaion HP-20", manufactured by Mitsubishi Chemical Ind. Ltd., Japan), and treated with 10 % ethanolic hydrogen chloride solution, and then crystallized from isopropanol-diethyl ether to give α-methyl-4-(α-hydroxyimino-3-pyridylmethyl)cinnamic acid hydrochloride (595 mg, yield: 93.3 %) as colorless crystals.

m.p. 239 -247°C. (decomp.)

Mass (m/e): 282 ($M^+$)

IR (Nujol) $cm^{-1}$: 3080-3300, 2500-2800, 1688.

Reference Example 1

Magnesium (146 mg) is suspended in tetrahydrofuran (8 ml), and thereto is added dibromethane (3 drops) under argon stream. To the suspension is added dropwise a solution of p-bromobenzaldehyde diethylacetal (780 mg) in tetrahydrofuran (2 ml), and the mixture is refluxed with stirring for one hour to prepare a Grignard reagent. To the reagent thus prepared is added dropwise a solution of 3-formylpyridine (215 mg) in tetrahydrofuran (2 ml) with stirring under cooling, and the mixture is stirred at room temperature for 2 hours. After the reaction, benzene is added to the reaction mixture, and the mixture is treated with 10 % aqueous ammonium chloride solution. The organic layer is separated, washed with saturated aqueous saline solution, and dried. The organic layer is concentrated

under reduced pressure. The residue is treated with 10 % ethanolic hydrogen chloride solution and crystallized from ethanol-diethyl ether. The crystals (hydrochloride) are treated with a saturated aqueous sodium carbonate solution, and extracted with chloroform. Chloroform is distilled off under reduced pressure, and the resulting crystalline residue is recrystallized from benzene—n-hexane to give 4-(α-hydroxypyridylmethyl)benzaldehyde (295 mg, yield: 69.2 %) as pale yellow prisms.

m.p. 101 - 105°C.

Mass (m/e): 213 ($M^+$)

IR (Nujol) $cm^{-1}$: 3150, 1695.

CLAIMS:

1. A cinnamic acid derivative of the formula:

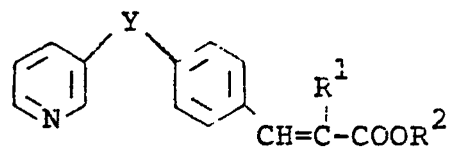

(I)

wherein $R^1$ is a lower alkyl group, $R^2$ is hydrogen atom or a lower alkyl group, and Y is hydroxymethylene, a lower alkoxymethylene, aminomethylene, hydroxyiminomethylene, a halogenomethylene or carbonyl group, or a pharmaceutically acceptable salt thereof.

2. The compound according to claim 1, wherein $R^1$ is methyl, $R^2$ is hydrogen atom or ethyl, and Y is hydroxymethylene, ethoxymethylene, aminomethylene, hydroxyiminomethlene, chloromethylene or carbonyl.

3. The compound according to claim 1, wherein $R^1$ is methyl, $R^2$ is hydrogen atom, and Y is hydroxymethylene or ethoxymethylene.

4. The compound according to claim 1, which is α-methyl-4-(α-hydroxy-3-pyridylmethyl)cinnamic acid.

5. The compound according to claim 1, which is sodium α-methyl-4-(α-ethoxy-3-pyridylmethyl)cinnamate.

6. A process for preparing a cinnamic acid derivative of the formula:

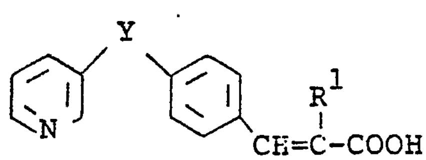

wherein $R^1$ is a lower alkyl group, Y is hydroxymethylene, a lower alkoxymethylene, aminomethylene, hydroxyiminomethylene, a halogenomethylene or carbonyl group, or a pharmaceutically acceptable salt thereof, which comprises hydrolyzing a compound of the formula:

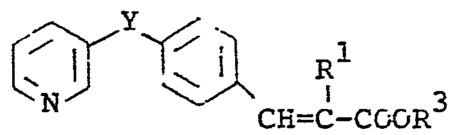

(II)

wherein $R^3$ is a lower alkyl, and $R^1$ and Y are as defined above, and optionally converting the product into a pharmaceutically acceptable salt thereof.

7. A process for preparing a cinnamic acid derivative of the formula:

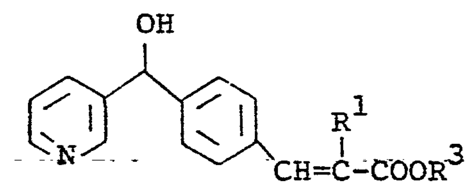

wherein $R^1$ is a lower alkyl group, and $R^3$ is a lower alkyl group, or a pharmaceutically acceptable salt thereof, which comprises reacting a compound of the formula:

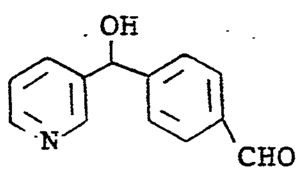

with a compound of the formula:

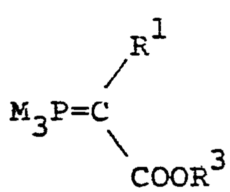

wherein M is phenyl, and $R^1$ and $R^3$ are as defined above, and optionally converting the product into a pharmaceutically acceptable salt thereof.

8. A process for preparing a cinnamic acid derivative of the formula:

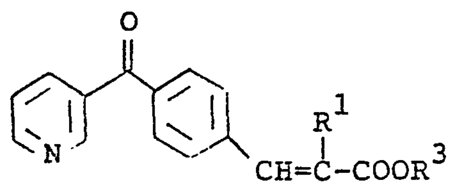

wherein $R^1$ is a lower alkyl group, and $R^3$ is a lower alkyl group, and a pharmaceutically acceptable salt thereof, which comprises oxidizing a compound of the formula:

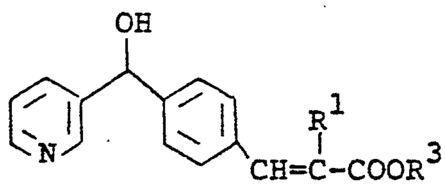

wherein $R^1$ and $R^3$ are as defined above, and optionally converting the product into a pharmaceutically acceptable salt thereof.

9. A process for preparing a cinnamic acid derivative of the formula:

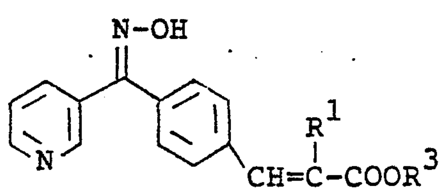

wherein $R^1$ is a lower alkyl group, and $R^3$ is a lower alkyl group, or a pharmaceutically acceptable salt thereof, which comprises reacting a compound of the formula:

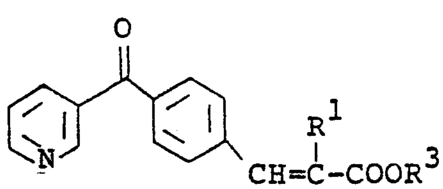

wherein $R^1$ and $R^3$ are as defined above, with hydroxylamine,

and optionally converting the product into a pharmaceutically acceptable salt thereof.

10. A process for preparing a cinnamic acid derivative of the formula:

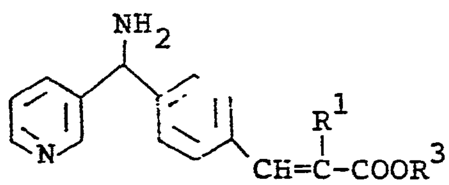

wherein $R^1$ is a lower alkyl group, and $R^3$ is a lower alkyl group, or a pharmaceutically acceptable salt thereof, which comprises reducing a compound of the formula:

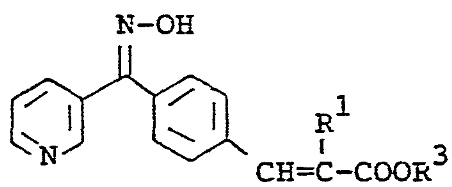

wherein $R^1$ and $R^3$ are as defined above, and optionally converting the product into a pharmaceutically acceptable salt thereof.

11. A process for preparing a cinnamic acid derivative of the formula:

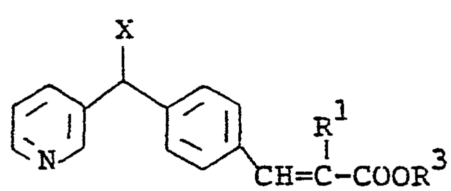

wherein $R^1$ is a lower alkyl group, $R^3$ is a lower alkyl group, and X is a halogen atom, or pharmaceutically acceptable salt thereof, which comprises reacting a compound of the formula:

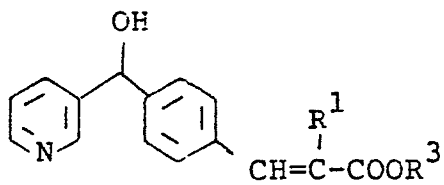

wherein $R^1$ and $R^3$ are as defined above, with a halogenation agent, and optionally converting the product into a pharmaceutically acceptable salt thereof.

12. A process for preparing a cinnamic acid derivative of the formula:

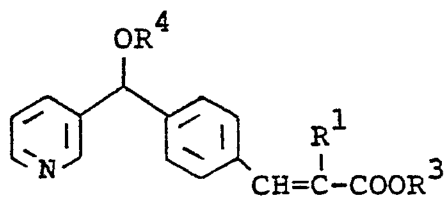

wherein $R^1$ is a lower alkyl group, $R^3$ is a lower alkyl group, and $R^4$ is a lower alkyl group, or a pharmaceutically acceptable salt thereof, which comprises reacting a compound of the formula:

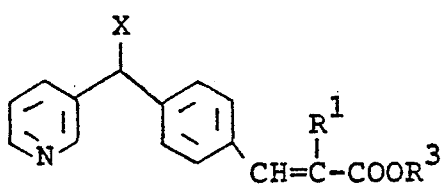

wherein X is a halogen atom, and $R^1$ and $R^3$ are as defined above, with a lower alkanol, and optionally converting the product into a pharmaceutically acceptable salt thereof.

13. A pharmaceutical composition which comprises a therapeutically effective amount of a compound of the formula:

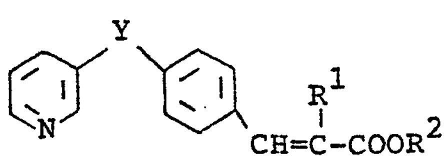

(I)

wherein $R^1$ is a lower alkyl group, $R^2$ is hydrogen atom or a lower alkyl group, and Y is hydroxymethylene, a lower alkoxymethylene, aminomethylene, hydroxyiminomethylene, a halogenomethylene or carbonyl group, or a pharmaceutically acceptable salt thereof and a pharmaceutically acceptable carrier therefor.

European Patent Office

# EUROPEAN SEARCH REPORT

0153678

Application number

EP 85 10 1715

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | FR-A-2 445 319 (ONO PHARMACEUTICAL CO., LTD. & KISSEI PHARMACEUTICAL CO., LTD.) * Page 47, line 34; page 48, lines 25,34; page 49, line 10; page 50, line 18; page 1 * | 1,7,13 | C 07 D 213/30<br>C 07 D 213/53<br>C 07 D 213/50<br>C 07 D 213/38<br>C 07 D 213/26<br>A 61 K 31/44 |
| D,A | JOURNAL OF MEDICINAL CHEMISTRY, vol. 24, no. 10, 1981, pages 1149-1155, Columbus, Ohio, US; T. TANOUCHI et al.: "Highly selective inhibitors of thromboxane synthetase. 2. Pyridine derivatives" * Pages 1150,1151 * | 1,7,13 | |
| | | | TECHNICAL FIELDS SEARCHED (Int Cl 4) |
| | | | C 07 D 213/00 |

The present search report has been drawn up for all claims

| Place of search | Date of completion of the search | Examiner |
|---|---|---|
| THE HAGUE | 06-05-1985 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82